# FASCICULE DE BREVET EUROPEEN

(11) **EP 3 197 357 B1**
(45) Date de publication et mention de la délivrance du brevet: **24.05.2023**
(21) Numéro de dépôt: 15780939.3
(22) Date de dépôt: 22.09.2015
(51) Int. Cl.: A61B 5/145, H01J 49/04, G01N 33/483, A61B 10/00

(54) **DISPOSITIF D'ANALYSE MOLECULAIRE IN VIVO EN TEMPS REEL**
VORRICHTUNG ZUR IN-VIVO-ECHTZEIT-MOLEKULARANALYSE
DEVICE FOR REAL-TIME IN VIVO MOLECULAR ANALYSIS

(30) Priorité: 22.09.2014 FR 1458925
(43) Date de publication de la demande: 02.08.2017
(73) Titulaire: Université de Lille, 59800 Lille (FR); Centre National de la Recherche Scientifique, 75016 Paris (FR)
(72) Inventeur: SALZET, Michel, F-59830 Bourghelles (FR); FOURNIER, Isabelle, F-59830 Bourghelles (FR); FOCSA, Cristian, F-59139 Wattignies (FR); ZISKIND, Michael, F-59000 Lille (FR); FATOU, Benoît, F-59221 Beauvin (FR); WISZTORSKI, Maxence, F-59152 Chereng (FR)
(74) Mandataire: Gevers & Orès
(86) Numéro de dépôt international: PCT/IB2015/057301
(87) Numéro de publication internationale: WO 2016/046748

(56) Documents cités:
- WO-A2-2014/079802
- DE-A1- 4 200 497
- US-A1- 2002 121 596
- US-A1- 2008 035 844
- US-A1- 2011 215 233
- US-B1- 6 358 243
- US-B1- 7 375 319

## Description

La présente invention concerne un dispositif d'analyse moléculaire in vivo en temps réel.

Le domaine de l'invention est celui de l'analyse de cellules d'un organisme vivant.

Le diagnostic précoce de pathologies est une étape cruciale pour les chirurgiens et les cliniciens. Le diagnostic doit conduire à prendre une décision claire le plus rapidement possible sur l'état physiopathologique du patient (humain ou animal). Cette étape doit être réalisée dans un laps de temps court et ceci avec un mimimum de dommages pour le patient et sans lui infliger des complications supplémentaires.

Depuis 25 ans, plusieurs outils de diagnostic non invasifs ont été développés, notamment l'imagerie par résonance magnétique, le scanner, la Tomographie par Emission de Positrons (TEP) ou la synographie. Ces techniques sont efficaces pour observer, localiser et déterminer la taille de régions anormales d'un point de vue tissulaire comme des régions cancéreuses. Certaines de ces techniques peuvent même donner des informations plus précises comme la production de nouveaux vaisseaux sanguins à l'intérieur de ces régions (néoangiogénèse) ou le catabolisme cellulaire.

Cependant, aucune de ces techniques n'a la capacité de donner des informations sur le contenu moléculaire de la région en cause. Cette information fait défaut notamment pour poser un diagnostic voir un pronostic de la pathologie. Une stratégie largement employée dans le cadre hospitalier est de procéder à l'excision de tissus (biopsie) dans la région anormale puis d'effectuer des analyses ex-vivo sur le tissu par différentes techniques et notamment histologiques (e.g. examen anatomopathologique) pour rechercher des caractéristiques morphologiques, cellulaires, tissulaires ou moléculaires. Dans le cadre de cancers, une telle pratique permet de confirmer la présence de tumeurs malignes et d'en obtenir la classification histologique (type, grade). D'autres techniques plus ciblées peuvent être mises en oeuvre pour obtenir un diagnostic telles que l'immunohistochimie (IHC) ou les techniques de PCR afin de rechercher des marqueurs spécifiques de la pathologie ou des mutations particulières.

Bien que cette stratégie soit largement utilisée, elle peut s'avérer longue, laissant durant ce temps le patient au bloc opératoire en attente de son diagnostic. Il existe donc un intérêt réel au dévelopement de techniques permettant le recueil d'informations moléculaires in-vivo. Le dispositif recherché doit permettre d'obtenir ces informations in-vivo mais également en temps réel pendant le temps de présence dans le bloc opératoire.

Parmis les techniques qui peuvent permettre l'obtention d'informations moléculaires in-vivo il faut s'orienter vers les techniques spectroscopiques. Les spectroscopies Raman, IR ou de fluorescence sont des techniques répondant à ces critères. Toutefois, ces techniques présentent certains inconvénients que sont soit la nécessité d'utiliser un traceur pour visualiser la région d'intérêt, soit de recueillir des profils complexes (i.e. chaque molécule présente dans la région analysée donne un spectre complexe et le spectre de la région cellulaire analysée est une superposition de l'ensemble des spectres des molécules composant la région) ne permettant pas toujours d'observer des variations moléculaires entre une zone normale et pathologique et requérant l'utilisation de traitements statistiques extrêmement complexes.

En revanche, une autre méthode spectroscopique, la spectrométrie de masse, peut répondre à ce besoin de diagnostic rapide in-vivo en temps-réel. La spectrométrie de masse est une technique basée sur la mesure du poids moléculaire des espèces. Conventionnellement la mesure de masse est effectuée suivant le schéma, création d'ions en phase gazeuse à partir de l'échantillon (in-vitro) par la source de production d'ions de l'instrument, séparation des ions formées en fonction de la quotité m/z dans la partie analyseur puis détection du courant ionique. Les échantillons analysés peuvent être solide, liquide ou gazeux. Cependant, la source d'ions utilisée sera adaptée à l'état dans lequel se trouve l'échantillon. A partir de mélanges complexes, la spectrométrie de masse offre l'avantage de permettre l'observation d'un signal pour chaque espèce puisque celles-ci sont séparées en fonction de m/z sauf si les composés ont la même formule brute ou si les performances de l'instrument sont insuffisantes. Historiquement la spectrométrie de masse a conduit à l'avènement de différentes technologies pour les sources de production d'ions et les analyseurs, les sources et analyseurs étant combinables entre-eux de différentes façons permettant de créer des instruments de caractéristiques définies en termes de composés analysables, d'état de l'échantillon, de performances instrumentales. Plus récemment, les techniques de spectrométrie de masse ont évolué pour permettre de passer de l'analyse d'extraits *in vitro* à l'analyse d'organismes ou de parties d'organismes ex-vivo. Le développement de ces techniques a contribué à l'avènement d'un nouveau domaine de recherche intitulé Imagerie par Spectrométrie de Masse. Actuellement, les sources de productions d'ions les plus couramment utilisées car compatibles dans ce domaine sont les sources dites de Spectrométrie de Masse d'Ions secondaires (Secondary Ion Mass Spectrometry, SIMS), les sources de Désorption/lonisation Laser (Laser Desorption lonization, LDI), les sources de Désorption Ionisation/Laser Assistées par Matrice (Matrix Assisted Laser Desorption/Ionization, MALDI), les sources de Désorption/lonisation par Electronéblusation (Desorption ElectroSpray lonization, DESI) et les sources d'Ablation Laser/ Plasma Induit (Laser Ablation - Inductively Coupled Plasma, LA-ICP). Ces technologies telles quelles permettent l'analyse d'organismes ou partie d'organismes ex-*vivo* pour lesquelles elles permettent la caractérisation moléculaire mais elles ne sont pas employables *in-vivo* sur des organismes vivants.

En effet, dans le cas du DESI « pour Desorption Electrospray lonization » en anglais, un jet de goutellettes de solvent chargées produites par un processus d'électronébulisation est dirigé sur la surface de l'échantillon. Les goutellettes vont rebondir à la surface de l'échantillon avec un processus de capture de molécules de surface lors de la phase d'interaction des goutellettes avec la surface.. Les molécules sont aspirées par un capillaire à destination de l'entrée du spectromètre de masse. La source DESI a prouvé ses capacités pour de multiples échantillons biologiques comme des tissus ou des organes. Ceci est illustré dans l'article de Calligaris D et al, 2013, J Mass Spectrom, 48 (II), 1178-87 où le DESI est combiné à l'imagerie conventionnelle *in vivo.* Une modification instrumentale a été effectuée pour tenter l'utilisation du DESI in vivo : Chen CH et al, 2013, Anal. Chem 85(24), 11843-50. Dans ce cas, un jet de solvant sous pression est dirigé sur le tissu. Ce jet est positionné à l'intérieur d'un tube de transfert permettant d'assurer le transport des molécules chargées générées par le jet vers le spectromètre de masse. Malgré son utilisation in vivo, cet instrument nécessite un contact avec la zone à analyser. L'analyse de la surface de façon continue peut induire des effets de contamination pour une caractérisation des tissus biologiques.

Afin de circonscrire ce problème, une solution est l'ablation laser comme procédé de désorption : Nemes P, Vertes A, 2007 Anal Chem., 79(21), 8098-106 - Park SG, Murray KK, 2011 J Am Soc Mass Spectrom 22(8), 1352-62.Une technique d'ablation laser suivie d'une capture des molécules ablatées par un jet de solvant d'électronébulisation, technique connue sous l'acronyme LAESI (pour « Laser Ablation ElectroSpray lonization » en anglais) a été introduite par la même équipe et l'équipe du Pr. K. Murray la même année (2007). L'ablation se fait avec un laser pulsé émettant dans le domaine de l'infrarouge. Les molécules ablatées sont ionisées par un jet d'électronébulisation et transférées vers l'entrée du spectromètre de masse. L'avantage consiste dans l'excitation de molécules biologiques abondantes comme l'eau avec une faible résolution spatiale. Cette technique a déjà été utilisée pour des organismes vivants. Cette technique est difficilement miniaturisable et nécessite l'emploi d'un solvant avec les inconvénients que cela comporte dans une optique d'utilisation in-*vivo.*

Les techniques invasives seront laisées sur le côté dans le cas du présent dispositif et notamment celles faisant appel à un bistouri quelqu'en soir la nature (manuel, électrique,...).

Le document US 2010/0012831 enseigne une méthode d'imagerie moléculaire 3D ex-vivo basée sur la technique LAESI. Dans la méthode LAESI un laser infrarouge est utilisé pour ablater les molécules. Les molécules ablatées sont capturées par un jet de goutelettes de solvant organique chargées produites par ionisation par Electronébulisation (ESI) puis acheminées vers l'analyseur de masse via une interface utilisant des champs électriques.Ici, la matière ablatée, pour être analysée, doit être capturée par un jet de goutellettes de solvant organique, ce qui n'est pas compatible avec une utilisation in vivo, de même que l'application d'un champ éléctrique.
Les dispositifs utilisés pour la technique de LAESI ne sont pas compatibles de part leur structure pour une utilisation in vivo car ils sont essentiellement conçus pour une utilisation ex vivo

Le document US 7,910,881 s'apparente au document précédent et présente une méthode d'analyse ex vivo basée sur la technique de LADC (Laser Ablation Droplet Capture). Ici l'échantillon est désorbé de la surface puis capturé dans un solvant placé dans un capillaire au-dessus du point d'ablation. L'échantillon à analyser est donc dissout dans un solvant avant d'être transféré au spectromètre de masse. Une analyse en temps réel n'est pas envisageable avec ce dispositif de par le temps que met l'échantillon à arriver à l'analyseur et des pertes de matières possibles au court du transfert.

Le document US 2012/0156712 propose un système d'analyse de tissus in vivo en temps réel. L'ablation du tissu à analyser est faite au moyen d'une électrode ou d'un bistouri électrique. Il s'agit donc d'une méthode invasive.

L'article « In Situ, Real-Time Identification of Biological Tissues by Ultraviolet and Infrared Laser Desorption lonization Mass Spectrometry » Anal. Chem. 2011, 83, 1632-40, traite d'une méthode d'analyse in situ de tissus biologiques dans le cadre de diagnostics ou de traitements chirurgicaux de divers types de cancers. Le dispositif est composé d'un laser couplé à un tube de transfert qui est relié à un spectromètre de masse via une source d'ionisation. En premier lieu cette source d'ionisation présente l'inconvénient d'endommager les molécules présentant un fort poids moléculaire. En second lieu, ce dispositif est encombrant de sorte que s'il permet l'analyse in situ, il n'est pas adapté à l'analyse in vivo. En troisième lieu, il ne peut pas fournir l'information pertinente en temps réel.

Le document DE 42 00 497 A1 divulgue un dispositif d'analyse d'échantillons plastiques ablatés par pyrolyse laser au moyen d'une sonde raccordée à un spectromètre de masse. Le spectromètre de masse est configuré pour atomiser les particules ablatées.

Le document WO 2014/079802 A2 divulgue un dispositif de spectrométrie de masse à plasma à couplage inductif d'Ablation (LA-ICP-MS) vaporisant et atomisant des particules abalatées au moyen d'un plasma d'argon porté à une température de 8000°C.

Le document US 2011/0215233 A1 divulgue un système permettant de mettre en oeuvre la technique d'ablation laser suivie d'une capture des molécules ablatées par un jet de solvant d'électronébulisation.

La présente invention a ainsi pour premier objet le développement d'un dispositif d'analyse de matériel biologique in vivo en temps réel basé sur la spectrométrie de masse.

Selon l'invention, un dispositif d'analyse moléculaire de matériel biologique comprend :
- un laser, éventuellement aidé d'un oscillateur paramétrique optique, configuré pour émettre une longueur d'onde comprise entre 2,5 µm et 12 µm, ledit laser ainsi configuré étant destiné à ablater ledit matériel biologique en éjectant des particules chargées et/ou non chargées ;
- un spectromètre de masse ; et
- une sonde comportant :
   - au moins une première fibre d'analyse, et
   - un tube de transfert,
ladite au moins première fibre d'analyse (A, 14) étant raccordée au laser (34), ledit tube de transfert étant raccordé au spectromètre de masse.

On dispose ainsi d'un instrument miniaturisé adapté à l'analyse in vivo.

On entend par analyse au sens de l'invention l'obtention de données moléculaires permettant de renseigner sur l'état physiologique d'un patient a un instant t (diagnostic) ou futur (pronostic). Ces données moléculaires pouvant être directement issues du patient mais également issus des organsimes symbiotiques du patient (virus, bactérie...)

La fibre d'analyse est raccordée à un laser d'ablation.

A titre d'exemple, la longueur d'onde du laser d'ablation peut-être comprise entre 2,8 µm et 3,2 µm. Plus généralement, le laser est configuré pour fournir, éventuellement aidé d'un oscillateur paramétrique optique (OPO), une longueur d'onde comprise entre 2,5 µm et 12 µm.

Le tube de transfert est raccordé à un spectromètre de masse.

Suivant une caractéristique additionnelle du dispositif, le laser est un laser impulsionnel configuré pour générer un fasiceau dont l'énergie est comprise entre 2mJ/impulsion et 15mJ/impulsion et la surface entre 30µm² et 3mm².Suivant une caractéristique additionnelle un système de focalisation et de transfert des ions est interposé entre ledit tube de transfert et le spectromètre de masse.

Suivant une autre caractéristique additionnelle, une grille métallique, avantageusement extrêmement fine, est introduite entre le tube de transfert et le spectromètre de masse.

Cette grille permet avantageusement d'augmenter la sensibilité d'analyse en augmentant la production d'ions.

Selon une autre caractéristique additionnelle de l'invention, un capillaire de nébulisation est raccordé au capillaire de dépréssurisation.

Ainsi, le capillaire de nébulisation est relié à un moyen de distribution de solvant.

Il peut s'avérer nécessaire de prévoir une deuxième fibre d'analyse.

Selon l'invention, le dispositif comporte de plus une fibre de thérapie pour la thérapie laser.

Avantageusement, la fibre de thérapie est raccordée à un laser de longeur d'onde adaptée pour détruire les cellules..

L'avantage du système est de permettre la combinaison d'un système d'analyse et de celui d'un système de thérapie. Suivant les résultats obtenus via la partie analytique, les tissus pourront être traités via la fibre de thérapie.

Suivant un mode de réalisation particulier, la sonde comporte de plus un canal d'illumination et un canal de prise de vue.

Ce mode de réalisation sera tout particulièrement adapté à une utilisation endoscopique

La présente invention apparaîtra maintenant de manière plus détaillée dans le cadre de la description qui suit d'un exemple de réalisation donné à titre illustratif en se référant aux figures annexées qui représentent :
- La figure 1, un schéma en perspective d'une sonde suivant un premier mode de réalisation de l'invention,
- La figure 2, un schéma en perspective d'une sonde endoscopique selon l'invention,
- La figure 3, un schéma de cette sonde raccordée aux équipements nécessaires à sa mise en oeuvre,
- la figure 4, un schéma de la ligne de transfert qui relie la sonde au spectromètre de masse,
- la figure 5, un diagramme de spectrométrie relatif à l'analyse de tissu biologique ex vivo et notamment de foie de boeuf, plus particulièrement :
   ∘ la figure 5a, le courant ionique total sur l'ensemble de la période d'acquisition
   o la figure 5b, représente le spectre obtenu pendant la période d'irradiation laser,
- la figure 6, une comparaison d'analyse ex vivo entre le foie et le cerveau d'un rat, plus particulièrement,
   ∘ la figure 6a, les résultats obtenus sur le foie,
   ∘ la figure 6b, les résultats obtenus sur le cerveau,
- la figure 7, une analyse d'une biopsie cancéreuse de lymphome de chien
- la figure 8, des analyses d'empreintes digitales, comparaison homme / femme (analyse in vivo sur le doigts)

Les éléments présents dans plusieurs figures sont affectés d'une seule et même référence.

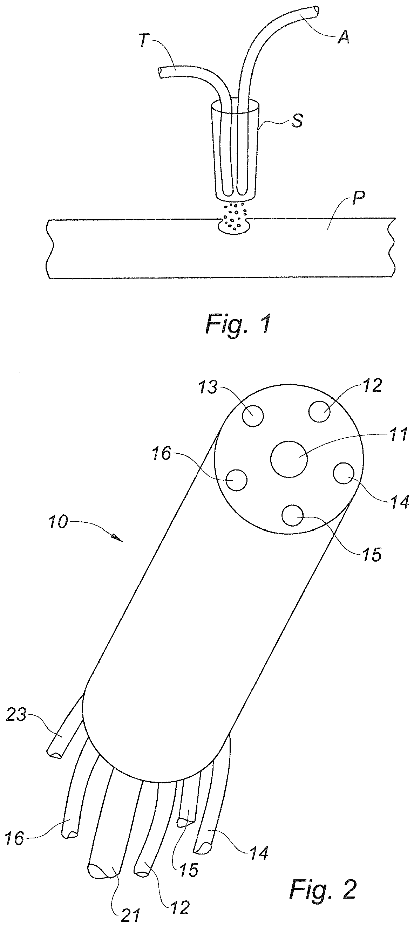

En référence à la figure 1, on a représenté une sonde S selon l'invention en quasi-contact avec un matériel biologique à analyser P, la peau d'un patient en l'occurrence (mais cela peut tout aussi bien être un organe).

La sonde S se présente comme un cylindre dans lequel figurent une fibre d'analyse A et un tube de transfert T. Ces deux éléments affleurent en face avant de la sonde, celle qui vient à proximité du matériel biologique à analyser. La fonction de ces éléments est explicitée plus loin.

Il s'agit là du mode de réalisation de base de l'invention qui permet d'effectuer une analyse externe sur la peau, les cheveux , les ongles ou interne sur un organe lors d'une chirurgie ouverte.

Suivant une évolution de l'invention la sonde est en fait une sonde endoscopique qui comporte des éléments additionnels.

En référence à la figure 2, la sonde endoscopique 10 se présente ici comme un cylindre présentant un évidemment axial 11.

Elle présente une face d'analyse ou face avant qui est visible sur la figure et elle présente aussi une face opposée, la face arrière qui n'apparaît pas sur la figure.

L'évidemment axial 11 est également cylindrique et un tube de transfert 21 y est inséré. La fonction de ce tube de transfert est détaillée plus loin.

Parallèlement à l'évidemment 11 sont disposés plusieurs éléments qui sont eux-aussi cylindriques.

En premier lieu, un canal d'illumination 12 tel qu'une fibre optique débouche sur la face arrière pour être raccordé à un dispositif d'éclairage non représenté sur cette figure.

En deuxième lieu, un canal de prise de vue 13 est disposé à proximité du canal d'illumination 12. Il comporte un appareil de prise de vue tel qu'une caméra et la sortie en face arrière se fait par une liaison vidéo 23.

En troisième lieu une première fibre optique d'analyse 14 qui affleure la face d'analyse débouche sur la face arrière. Son raccordement est explicité par la suite.

Avantageusement, on peut prévoir une seconde fibre optique d'analyse 15 qui débouche également sur la face arrière.

Pour le cas où la sonde est également utilisée pour le traitement par thérapie laser, celle-ci comporte encore une fibre de thérapie laser 16 qui débouche encore sur la face arrière.

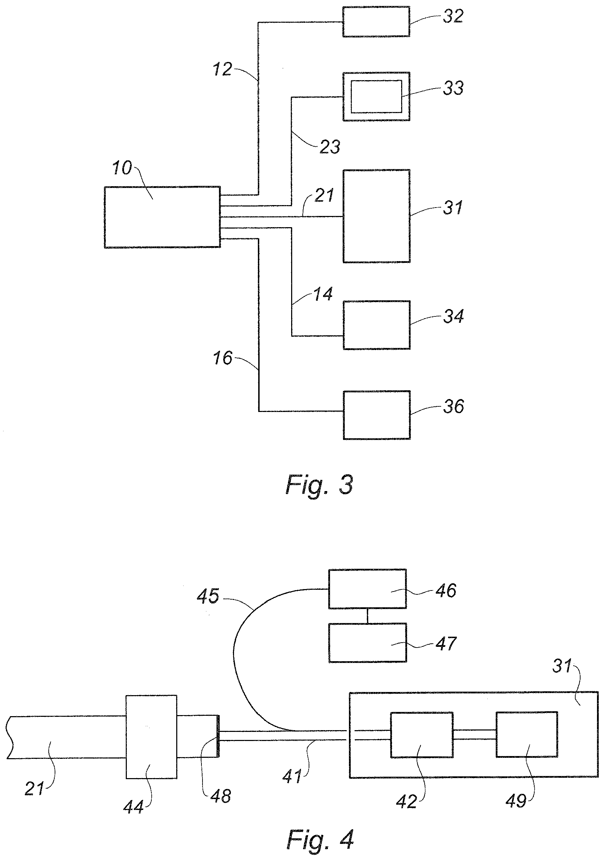

En référence à la figure 3, on explicite les différents raccordements de la sonde 10.

Le canal d'illumination 12 est raccordé à un dispositif d'éclairage 32.

La liaison vidéo 23 est raccordée à un écran de visualisation 33.

Le tube de transfert 21 est raccordé à un spectromètre de masse 31. Le détail de cette liaison est fourni plus loin.

Ce tube de transfert est de préférence constitué d'un matériau visant à minimiser les phénomènes d'absorption pour assurer un transfert efficace du matériel ablaté par la fibre d'analyse. Ce matériau est par exemple du PTFE.

La première fibre d'analyse 14 est raccordée à un premier laser d'ablation 34. Ce laser 34 a pour fonction d'échantilloner le tissu qu'il irradie provoquant ainsi l'éjection de particules chargées (ions moléculaires) et/ou non chargées en phase gazeuse.

La longueur d'onde de ce laser peut être choisie dans le domaine qui s'étend de l'infrarouge à l'ultraviolet, préférentiellement dans l'infrarouge.

Il s'agit par exemple d'un laser de longueur d'onde comprise entre 2,8µm et 3,2 µm, typiquement un laser Erbium-YAG émettant à une longueur d'onde de 2,94 µm.

On peut également citer :
- les lasers Neodym-YAG : 1,064 µm ; 0,532 µm ; 0,355 µm ; 0,266µm
- les lasers Xe-Ne : de 2 µm à 4 µm
- les lasers HF (Hydrogène Fluoride) : 2,6 µm
- les lasers à fibre de type Ytterbium, dopé au bismuth Thulium ou Holmium : de 1,07 µm à 2,1 µm

Ces lasers peuvent être utilisés comme sources directes d'émission ou couplés avec un OPO (Oscillateur Paramétrique optique). Un OPO permet en effet, à partir d'une onde laser longueur d'onde donnée de produire deux ondes de longueur d'onde plus importante. Cela permet donc d'élargir la gamme de longueur d'onde utilisable pour le laser considéré, vue par le matérial biologique à ablater.

De manière générale, on cherche à couvrir la gamme de longueurs d'onde allant de 2,5 µm à 12 µm. En effet, dans cette gamme de longueurs d'onde, on couvre les bandes d'absorption des liaisons O-H, N-H, C-H, C=O, C=N, C=C, C-O, C-N et C-C susceptibles d'être présentes dans le matérial biologique à ablater.

En particulier, dans cette gamme de longueurs d'onde allant de 2,5 µm à 12 µm, le laser permet d'ablater le matériel biologique en générant au moins des particules chargées, en particulier des ions moléculaires, en phase gazeuse.

On peut cependant se limiter à la gamme de longueurs d'onde comprise entre 2,5 µm et 3,5 µm. En effet, dans cette gamme de longueurs d'onde, on couvre les bandes d'absorption des liaisons O-H, N-H et C-H.

On peut se limiter à une gamme de longueurs d'onde plus restreinte, comprise entre 2,8pm et 3,2 µm. En effet, dans cette gamme de longueurs d'onde, on couvre les bandes d'absorption des liaisons O-H et N-H.

Par ailleurs, le laser d'ablation 34 sera avantageusement un laser impulsionnel configuré pour générer un faisceau dont l'énergie est avantageusement comprise entre 2mJ/impulsion et 15mJ/impulsion, la surface du faisceau (focalisation) étant comprise entre 30 µm² et 3mm².

L'énergie du faisceau pourra être comprise entre 5mJ/impulsion et 12mJ/impulsion, ou encore entre 5mJ/impulsion et 10mJ/impulsion, la surface du faisceau (focalisation) étant comprise entre 30 µm² et 3mm².

Pour les gammes d'énergies considérées précédemment, le faisceau laser pourra par ailleurs présenter une surface comprise entre 100 µm² et 3mm², entre 10⁻³ mm² et 3mm², entre 10⁻² mm² et 3mm², entre 10⁻¹ mm² et 3mm², entre 0,5mm² et 3mm², ou entre 0,5mm² et 2mm². En particulier, le faisceau du laser permet typiquement d'ablater un volume de matériel biologique dont la surface de base est de l'ordre du mm², ce qui correspond sensiblement à un faisceau dont la surface ou focalisation est également de l'ordre du mm².

Les inventeurs ont en effet pu constater que cette sélection dans l'énergie fournie par une impulsion du laser d'ablation 34 et dans la surface (focalisation) de ce faisceau assurait une meilleure production d'ions moléculaires et apportait donc un effet de synergie avec la sélection de la gamme de longueurs d'onde du laser précisée ci-dessus.

Il convient par ailleurs de noter que la profondeur de pénétration du faisceau laser est typiquement de quelques microns par impulsion laser.

Si l'on prévoit une deuxième fibre de diagnostic, celle-ci est raccordée à un deuxième laser d'ablation d'un type différent du premier. On augmente ainsi les possibilités du diagnostic. On citera à titre d'exemple le laser Neodym-YAG à une longueur d'onde de 0,532 µm. On citera également à titre d'exemple également un autre laser, éventuellement couplé à un OPO pour agir dans la gamme 2,5 µm à 3,5 µm. Ladite deuxième fibre d'analyse permettant avantageusement d'augmenter les possibilités d'analyse.

Les particules ainsi éjectées sont prises en charge par le tube de transfert 21 qui les achemine vers le spectromètre de masse 31.

La fibre de thérapie laser 16 est raccordée à un laser de thérapie 36. En effet, si l'analyse effectuée précédemment révèle que les tissus doivent être traités, le traitement peut avoir lieu immédiatement, ceci sans utiliser un quelconque équipement additionnel. Ainsi, on pourra par exemple utiliser, pour la thérapie, un laser (diode laser) émattenta à 980nm, comme proposé par Gonzalez-Mertinez & al., « Robot-assisted stereoactic laser ablation in medically intractable epilepsy : operative technique, Neurosurgery, 2014, suppl 2 : 167-172.

En référence à la figure 4, est détaillé un exemple de liaison entre la sonde 10 et le spectromètre de masse 31.

Le tube de transfert 21 se prolonge par un capillaire de dépressurisation 41 du côté opposé à celui de la sonde 10. Le capillaire est de préférence métallique. Il a un diamètre interne plus petit que celui du tube de transfert 21. Cela permet une augmentation de la dépressurisation du spectromètre de masse 31, ce qui induit une accélération des particules. Si ce capillaire 41 est métallique, on peut générer un champ électrique pour créer une différence de potentiel entre ce capillaire et l'entrée du spectromètre de masse 31.

Le capillaire de dépressurisation 41 se prolonge à l'entrée du spectromètre de masse 31 par un un système de focalisation et de transfert des ions 42 intégré à l'intérieur du spectromètre de masse.

En variante, ce système 42 peut être disposé à l'extérieur du spectromètre de masse, en amont de celui-ci, en référence au parcours de particules chargées ou non.

Par ailleurs, en variante également, ce système 42 peut être un système de focalication des ions ou un système de transfert des ions.

Ce système 42 permet de guider l'aérosol comportant des particules chargées ou non vers le spectromètre de masse.

Le tube de transfert 21 peut-être pourvu d'un moyen de chauffage 44 pour augmenter sa température.

On peut également prévoir un capillaire de nébulisation 45 qui vient se raccorder au capillaire de dépressurisation 41. Ce capillaire de nébulisation 45 est approvisionné par un distributeur de solvant 46. Un organe de contrôle 47 est prévu pour réguler le distributeur 46 de sorte que le débit de solvant soit celui souhaité. Ce solvant permet de reproduire un processus d'electronébulisation conventionnel afin d'augmenter le rendement de production de molécules chargées (ions moléculaires). L'avantage d'introduire le solvant à cet endroit est l'absence de toxicité tant vis-à-vis des utilisateurs que vis-à-vis des tissus biologiques.

Cependant, ceci n'est proposé qu'à titre optionnel. Aussi, on peut envisager qu'aucun moyen de nébulisation relié à un moyen de distribution de solvant n'est prévu entre le tube de transfert T, 21 et le spectromètre de masse 31. En effet, un avantage de l'invention est que le processus d'ablation du matériel biologique permet d'éjecter au moins des particules chargées (ions molcéulaires) en quantité suffisante pour leur analyse ultérieure par le spectromètre de masse.

Par ailleurs, on peut également prévoir un capillaire de distribution qui vient se se raccorder au capillaire de dépressurisation. Ce capillaire de distribution est alors approvisionné par un distributeur de gaz contenant des ions GH+. Ceci permet d'induire, par collision, un transfert de protons aux particules à analyser et ainsi d'augmenter le rendement de la production d'ions. Ce cas n'est pas représenté sur les figures annexées, mais l'implantation de ce capillaire de distribution et du ditributeur de gaz peut être similaire à celle du capillaire de nébulisation 45 et de son distributeur de solvant 46, respectivement.

Cependant, ceci n'est proposé qu'à titre optionnel. En effet, on peut envisager qu'aucun capillaire de distribution relié à un distributeur de gaz n'est prévu entre le tube de trasnfert T , 21 et le spectromètre de masse 31. En effet et pour rappel, un avantage de l'invention est que le processus d'ablation du matériel biologique permet d'éjecter au moins des particules chargées (ions molcéulaires) en quantité suffisante pour leur analyse ultérieure par le spectromètre de masse.

On peut également prévoir une grille métallique 48 dans la ligne de transfert qui va de la sonde 10 au spectromètre de masse 31.

Cette grille est par exemple disposée entre le tube de transfert 21 et le capillaire de dépressurisation 41, comme représenté sur la figure 4.

Il s'agit d'une grille extrêment fine du type de celles qui sont utilisées en microscopie électronique. Sa fonction est de casser les particules ou les aggrégats de particules éjectés par le laser d'ablation 34 pour qu'elles soient finalement accélérés à destination du spectromètre de masse 31. Cette grille 48 ne casse pas les molécules, dont certaines sont sous forme ionique dans le tube de transfert T, 21, mais des particules plus grosses.

Un spectromètre de masse comprend de manière classique et dans l'ordre suivant :
- une source
- un système de transfert et de focalisation des ions
- au moins un analyseur de masse

Il comprend également un système de détection.

Selon un mode de réalisation particulier de l'invention, le spectromètre de masse ne comporte pas de source et comprend au moins un système de focalisation et/ou de transfert des ions interposé entre le tube de transfert et ledit analyseur de masse.

De manière non limitative, on peut citer à titre d'exemples de systèmes de focalisation et/ou de transfert des ions, un capillaire de transfert, un écrémeur, une lentille de focalisation, un système de transfert à champs multipolaires, un *ion tunnel,* une lentille électrostatique.

Le spectromètre de masse peut également comporter des éléments visant à améliorer ses performances tels que par exemple un système de mobilité ionique

Selon un mode de réalisation possible, le système de focalisation et de transert des ions est un capillaire de transfert.

Le spectromètre comprend un analyseur de masse 49. L'analyseur de masse employé peut-être de tout type qu'il soit simple (eg. Secteur magnétique simple (B) ou à double focalisation (BE, EB), Quadripôle (Q), Trappe ionique (TI), temps de vol (TOF), résonance d'ions cyclotronique (ICR), orbitrappe), combiné (eg Triple Quadripole) ou hybride (eg Q-orbitrappe, Q-TOF).

En variante, l'analyseur de masse employé peut être un autre système de dséparation des ions (e.g. mobilité ionique).

On aborde maintenant la stratégie envisagée lors de l'utilisation de la présente invention.

Le dispositif selon l'invention fonctionne sur la base d'un processus d'ablation laser permettant d'échantillonner le matériel biologique à analyser. Cela conduit à l'éjection de particules en phase gazeuse (chargées ou non). La matière ablatée est remise en temps réel au spectromètre de masse 31 via le tube de transfert 21. En effet, le processus d'ablation et l'éjection de particules, liée à cette ablation, ainsi que le temps de transit dans le tube de transfert sont courts et peuvent être qualifiés de temps réel. Cela permet de collecter des profils moléculaires (signaux provenant de l'analyse du matériel biologique correspondant à des biomolécules de type composés organiques, acides aminés, métabolites, lipides, peptides...) caractéristiques de la zone analysée.

Avantageusement, ces profils seront comparés à une banque de données de profils moléculaires obtenus par l'utilisation du présent dispositif, en temps réel permettant d'obtenir des informations de manière rapide.

La banque de données moléculaires est établie en utilisant le présent dispositif de manière *ex-vivo* sur des biopsies de patients représentant différents grades et stades de la pathologie considérée. Une cohorte d'échantillons de patients sains ou non est également intégrée à la banque de données.

On peut cependant établir la banque de données autrement.

Selon une utilisation particulière, le chirurgien déplacera la sonde à la surface ou à l'intérieur du patient sur le matériel biologique concerné pour savoir si il se situe dans une zone cancéreuse ou non lui permettant d'envisager de manière rapide un traitement pour le patient et notamment les zones qu'il aura à retirer chirurgicalement. Ces zones à retirer pouvant avantageusement être retirées grâce à la fibre de thérapie selon un mode de réalisation particulier du dispositif selon l'invention.

L'invention a permis d'obtenir les résultats suivants.

### Résultat 1 : Analyse de tissus biologiques ex vivo

Un laser émettant des impulsions nanosecondes à une fréquence de 10Hz (Quantel Easy Brillant, Les Ulis, France), connecté à un système OPO avec un cristal de type LiNbO₃ (variable en longueur d'onde entre 2,5 et 4,5µm, LaserSpec, Malonne, Belgique) réglé à une longueur d'onde de 2940nm est utilisé. Un tube de transfert en Téflon (10mm diamètre interne) est utilisé pour le transfert des particules chargées et non chargées, et est connecté de manière directe à un spectromètre de masse de type trappe ionique dont la source a été retirée (HCT Ultra, Bruker Daltonics, Brême, Allemagne). L'arrivée de N2 du spectromètre de masse a été déconnectée pour permettre l'ajout d'une pompe visant à augmenter le débit d'aspiration dans le tube de transfert. L'analyse des composés issu de l'irradiation laser est effectué par le spectromètre de masse en mode négatif sur une gamme de rapport masse sur charge (m/z) comprise entre 150 et 1000.

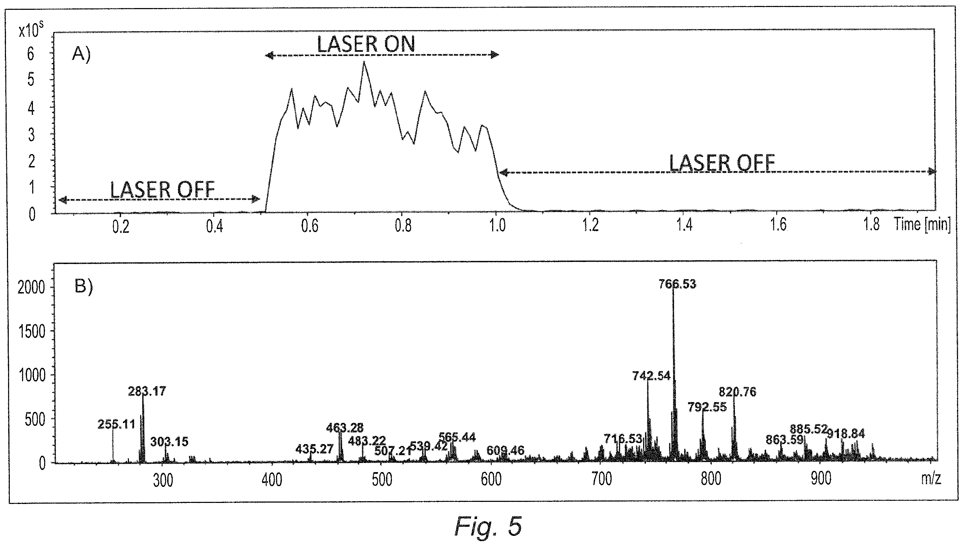

La première expérience présentée en Figure 5 est l'analyse ex *vivo* d'un morceau de foie de boeuf. Sur celui-ci est réalisée une irradiation de 7mJ/tir laser sur une zone d'1mm² (1 tir laser = 1 impulsion laser). Un nombre de 3 phases a été choisi lors de l'étape de l'acquisition : une première étape d'absence d'irradiation laser, une phase d'irradiation laser et une autre phase d'absence d'irradiation laser. La Figure 5A représente le courant ionique total sur l'ensemble de la période d'acquisition et la Figure 5B le spectre obtenu durant la période d'irradiation laser. Le courant ionique total montre que la présence de signal détecté est en corrélation avec l'irradiation laser. Il n'y a donc pas de composés qui adhèrent à la paroi interne du tube de transfert et cela montre une analyse rapide, en temps réel. Typiquement, le temps d'analyse est inférieur à la seconde.

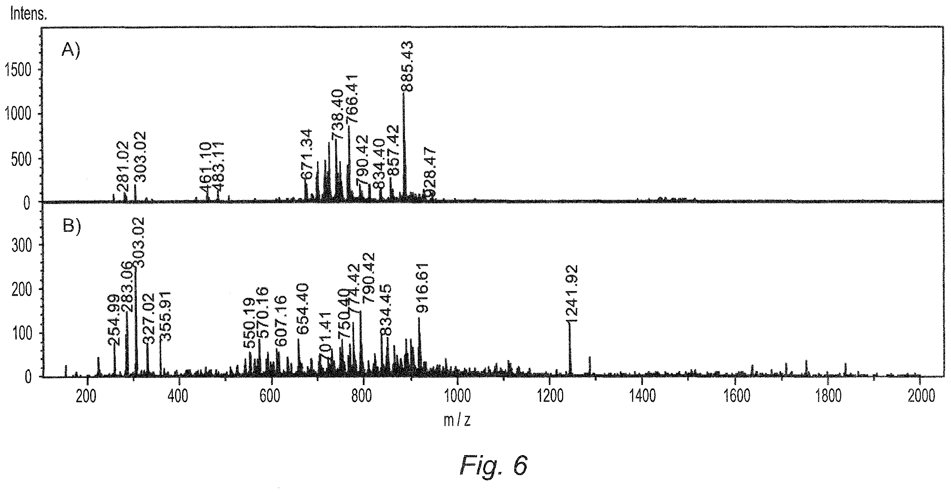

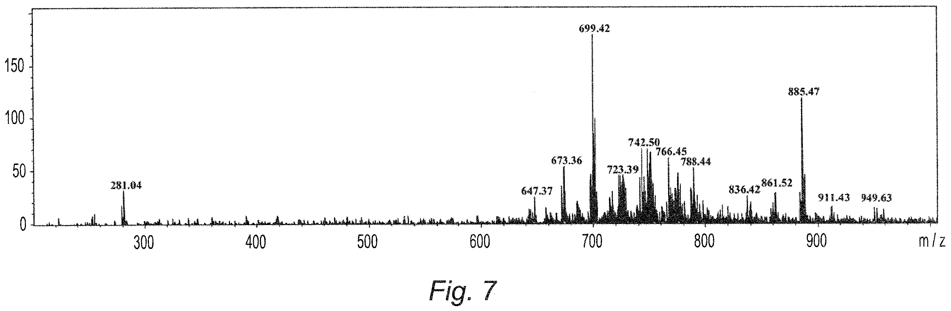

Sur la Figure 6 une comparaison de 2 organes chez le rat, le cerveau et le foie, est effectuée sur une acquisition sur chaque organe. Une irradiation de 30 secondes à 7mJltir laser sur une région de chaque organe. La Figure 6 montre une différence de rapport m/z entre ces 2 organes ce qui montre une spécificité de composition moléculaire du cerveau en comparaison avec le foie de rat. Sur la Figure 7, une analyse d'une biopsie cancéreuse de lymphome de chien est réalisée. Une irradiation de 30 secondes à 7mJ/tir laser est effectuée sur une région de cette biopsie. Le spectre enregistré est sélectionné au niveau de la période d'irradiation laser. Il montre une génération de signal correspondant à des lipides et à des acides gras et quelques signaux attribuables à des peptides. La présente invention permet d'effectuer des analyses en temps réel sans contamination au niveau de la paroi du tube en Téflon mais aussi entre des organes différents provenant d'un même animal. L'analyse en temps réel effectuée sur les différents organes montre un nombre important de signaux qui peuvent correspondre à des acides gras, des métabolites et des lipides. En référence à la Figure 6, la présente invention a la capacité de détecter des profils différents en fonction des organes étudiés et de leur statut physiologique (eg. sain vs cancéreux). L'avantage de cette analyse est une grande disparité des familles de molécules détectées qui peut ajouter une valeur significative à la fois sur la banque de données de profils moléculaires mais aussi sur la caractérisation des tissus biologiques.

### Résultat 2 : Analyse de tissus biologiques in vivo

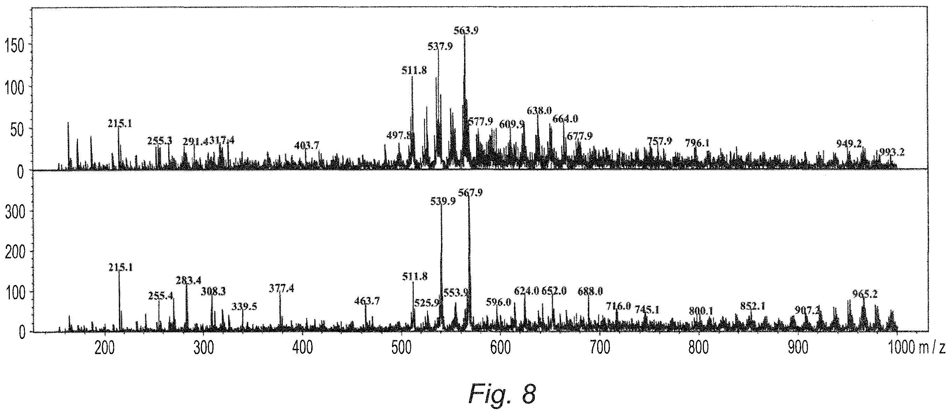

Sur la Figure 8 une analyse *in-vivo* en temps-réel des tissus de la peau d'individus de sexe différent est effectuée au niveau des doigts. Une irradiation de 10 secondes à 9mJ/tir laser (1 tir laser = 1 impulsion laser) pour chaque individu est réalisée grâce à un laser émettant des impulsions nanosecondes à une fréquence de 10Hz (Quantel Easy Brillant, Les Ulis, France), connecté à un système OPO avec un cristal de type LiNbO₃ (variable en longueur d'onde entre 2,5 et 4,5µm, LaserSpec, Malonne, Belgique) réglé à une longueur d'onde de 2940nm. Un tube de transfert en Téflon (10mm diamètre interne) est utilisé pour le transfert des particules chargées et non chargées, et est connecté de manière directe à un spectromètre de masse de type trappe ionique dont la source a été retirée (HCT Ultra, Bruker Daltonics, Brême, Allemagne). L'arrivée de N2 du spectromètre de masse a été déconnectée pour permettre l'ajout d'une pompe visant à augmenter le débit d'aspiration dans le tube de transfert. L'analyse des composés issu de l'irradiation laser est effectué par le spectromètre de masse en mode négatif sur une gamme de rapport masse sur charge (m/z) comprise entre 150 et 1000. Un nombre de 3 phases a été choisi lors de l'étape de l'acquisition : une première phase d'absence d'irradiation laser, une phase d'irradiation laser et une autre phase d'absence d'irradiation laser. Une distinction des différents individus est visualisée en Figure 8. La présente invention permet d'effectuer des analyses *in vivo* en temps réel sur des individus et de pouvoir observer des profils moléculaires spécifiques selon le sexe de l'individu. Cette analyse *in vivo* sur des individus montre l'effet non invasif et indolore de la présente invention pendant plusieurs secondes d'irradiation.

Un autre avantage de la présente invention est l'effet non invasif sur des organes lors de l'irradiation même de plusieurs dizaines de secondes sur un même point. Basé sur le profil moléculaire, la présente invention est utilisée pour la différenciation de zones très réduites (diamètre de la zone d'irradiation de 400µm) de tissus biologiques.

L'invention concerne également un procédé d'analyse moléculaire de matériel biologique, caractérisé en ce qu'il comprend les étapes suivantes :
- émettre un faisceau laser à une longueur d'onde comprise entre 2,5 µm et 12 µm en direction d'un matériel biologique, l'interaction entre ce faisceau et le matérial biologique provoquant l'ablation de ce dernier et l'éjection au moins de particules chargées, notamment d'ions moléculaires ;
- diriger le matériel biologique ablaté comportant au moins lesdites particules chargées vers un spectromètre de masse, par l'intermédiaire d'un tube de transfert ; et
- analyser la composition du matériel biologique ablaté dans le spectromètre de masse.

Ce procédé d'analyse moléculaire de matériel biologique pourra être réalisé *in vivo.*

Le faisceau laser pourra présenter une longueur d'onde comprise entre 2,5 µm et 3,5 µm, ou entre 2,8 µm et 3,2 µm.

Par ailleurs, le faisceau laser pourra être impulsionnel et présenter une énergie comprise entre 2mJ/impulsion et 15mJ/impulsion, la surface du faisceau (focalisation) étant comprise entre 30 µm² et 3mm².

L'énergie du faisceau pourra être comprise entre 5mJ/impulsion et 12mJ/impulsion, ou encore entre 5mJ/impulsion et 10mJ/impulsion, la surface du faisceau (focalisation) étant comprise entre 30 µm² et 3mm².

Pour les gammes d'énergies considérées précédemment, le faisceau laser pourra par ailleurs présenter une surface comprise entre 100 µm² et 3mm², entre 10⁻³ mm² et 3mm², entre 10⁻² mm² et 3mm², entre 10⁻¹ mm² et 3mm², entre 0,5mm² et 3mm², ou entre 0,5mm² et 2mm². En particulier, le faisceau du laser permet typiquement d'ablater un volume de matériel biologique dont la surface de base est de l'ordre du mm², ce qui correspond sensiblement à un faisceau également de l'ordre du mm².

De plus, ce procédé pourra prévoir une étape de chauffage du matériel biologique ablaté.

Ce procédé pourra également prévoir une étape au cours de laquelle le matérial biologique ablaté est tamisé par une grille, par exemple métallique.

Enfin, il convient de noter que l'invention propose également l'utilisation d'un dispositif d'analyse moléculaire conforme à l'invention pour ablater ledit matériel biologique en éjectant des particules chargées et/ou non chargées, et plus spécialement pour ablater ledit matériel biologique en éjectant au moins des particules chargées, notamment des ions moléculaires.

## Revendications

1. Dispositif d'analyse moléculaire de matériel biologique comprenant:
- un laser (34), éventuellement aidé d'un oscillateur paramétrique optique (OPO), configuré pour émettre une longueur d'onde comprise entre 2,5 µm et 12 µm, ledit laser (34) ainsi configuré étant destiné à ablater ledit matériel biologique en éjectant des particules chargées et/ou non chargées ;
- un spectromètre de masse (31) ; et
- une sonde (S, 10) comportant :
• au moins une première fibre d'analyse (A, 14), et
• un tube de transfert (T, 21),
ladite au moins première fibre d'analyse (A, 14) étant raccordée au laser (34), ledit tube de transfert étant raccordé au spectromètre de masse (31).

2. Dispositif selon la revendication 1, **caractérisé en ce que** le laser (34), éventuellement aidé d'un oscillateur paramétrique optique (OPO), est configuré pour émettre une longueur d'onde comprise entre 2,5 µm et 3,5 µm.

3. Dispositif selon l'une quelconque des revendications précédentes, **caractérisé en ce que** le laser (34), éventuellement aidé d'un oscillateur paramétrique optique (OPO), est configuré pour émettre une longueur d'onde comprise entre 2,8 µm et 3,2 µm.

4. Dispositif selon l'une quelconque des revendications précédentes, **caractérisé en ce que** le laser (34) est un laser impulsionnel configuré pour générer un faisceau dont l'énergie est comprise entre 2mJ/impulsion et 15mJ/impulsion et dont la surface est comprise entre 30 µm² et 3mm².

5. Dispositif selon l'une quelconque des revendications précédentes, **caractérisé en ce que** le laser (34) est un laser impulsionnel configuré pour générer un faisceau dont l'énergie est comprise entre 5mJ/impulsion et 12mJ/impulsion, par exemple entre 5mJ/impulsion et 10mJ/impulsion et la surface est comprise entre 30 µm² et 3mm².

6. Dispositif selon l'une des revendications 4 ou 5, **caractérisé en ce que** le laser (34) est configuré pour générer un faisceau dont la surface est comprise entre 0,5mm² et 2mm².

7. Dispositif selon l'une des revendications précédentes, **caractérisé en ce qu'**un système (42) de focalisation et/ou de transfert, en l'occurrence des ions moléculaires destinés à être formés lors d'une ablation de matériel biologique, est interposé entre ledit tube de transfert (T, 21) et le spectromètre de masse (31).

8. Dispositif selon la revendication précédente, **caractérisé en ce que** ledit système (42) de focalisation et/ou de transfert est un capillaire de transfert.

9. Dispositif selon l'une quelconque des revendications précédentes, **caractérisé en ce que** ledit tube de transfert (T, 21) est muni d'un moyen de chauffage (44).

10. Dispositif selon l'une quelconque des revendications 6 à 8, **caractérisé en ce qu'**un capillaire de dépressurisation (41) est inséré entre ledit tube de transfert (T, 21) et ledit système (42) de focalisation et/ou de transfert, le diamètre intérieur de ce capillaire de dépressurisation (41) étant inférieur à celui du tube de transfert (T, 21).

11. Dispositif selon la revendication précédente, **caractérisé en ce que** le capillaire de dépressurisation (41) est métallique et **en ce qu'**il est prévu un moyen pour générer une différence de potentiel entre ledit capillaire de dépressurisation (41) et le spectromètre de masse (31).

12. Dispositif selon l'une quelconque des revendications précédentes, **caractérisé en ce qu'**une grille métallique (48) est introduite entre ledit tube de transfert (T, 21) et ledit spectromètre de masse (31).

13. Dispositif selon l'une quelconque des revendications 10 à 12, **caractérisé en ce qu'**un capillaire de nébulisation (45) est raccordé audit capillaire de dépressurisation (41) et par ailleurs relié à un moyen de distribution de solvant (46).

14. Dispositif selon l'une quelconque des revendications 1 à 12, **caractérisé en ce qu'**aucun capillaire de nébulisation relié à un moyen de distribution de solvant n'est prévu entre le tube de transfert (T, 21) et le spectromètre de masse (31).

15. Dispositif selon l'une quelconque des revendications 1 à 13, **caractérisé en ce qu'**un capillaire de distribution est raccordé audit capillaire de dépressurisation et par ailleurs relié à un distributeur de gaz.

16. Dispositif selon l'une des revendications 1 à 14, **caractérisé en ce qu'**aucun capillaire de distribution relié à un distributeur de gaz n'est prévu entre le tube de transfert (T , 21) et le spectromètre de masse (31).

17. Dispositif selon l'une quelconque des revendications précédentes, **caractérisé en ce que** ladite sonde (S, 10) comporte une deuxième fibre d'analyse (15).

18. Dispositif selon l'une quelconque des revendications précédentes, **caractérisé en ce que** ladite sonde (S, 10) comporte de plus une fibre de thérapie (16).

19. Dispositif selon la revendication précédente, **caractérisé en ce que** ladite fibre de thérapie (16) est raccordée à un laser de thérapie (36).

20. Dispositif selon l'une quelconque des revendications précédentes, **caractérisé en ce que** ladite sonde (S, 10) comporte de plus un canal d'illumination (12) et un canal de prise de vue (13).

## Patentansprüche

1. Vorrichtung zur molekularen Analyse von biologischem Material, umfassend:
- einen Laser (34), gegebenenfalls unterstützt durch einen optischen parametrischen Oszillator (OPO), der konfiguriert ist, um eine Wellenlänge im Bereich zwischen 2,5 µm und 12 µm zu emittieren, wobei der somit konfigurierte Laser (34) dazu bestimmt ist, das biologische Material durch Ausstoßen von geladenen und/oder ungeladenen Teilchen abzutragen;
- ein Massenspektrometer (31); und
- eine Sonde (S, 10), die aufweist:
• mindestens eine erste Analysefaser (A, 14) und
• ein Übertragungsrohr (T, 21),
wobei die mindestens eine erste Analysefaser (A, 14) an den Laser (34) angeschlossen ist,
wobei das Übertragungsrohr an das Massenspektrometer (31) angeschlossen ist.

2. Vorrichtung nach Anspruch 1, **dadurch gekennzeichnet, dass** der Laser (34), gegebenenfalls unterstützt durch einen optischen parametrischen Oszillator (OPO), konfiguriert ist, um eine Wellenlänge im Bereich zwischen 2,5 µm bis 3,5 µm zu emittieren.

3. Vorrichtung nach einem der vorstehenden Ansprüche, **dadurch gekennzeichnet, dass** der Laser (34), gegebenenfalls unterstützt durch einen optischen parametrischen Oszillator (OPO), konfiguriert ist, um eine Wellenlänge im Bereich zwischen 2,8 µm und 3,2 µm zu emittieren.

4. Vorrichtung nach einem der vorstehenden Ansprüche, **dadurch gekennzeichnet, dass** der Laser (34) ein Impulslaser ist, der konfiguriert ist, um einen Strahl zu erzeugen, dessen Energie im Bereich zwischen 2 mJ/Impuls und 15 mJ/Impuls liegt und dessen Oberfläche im Bereich zwischen 30 µm² und 3 mm² liegt.

5. Vorrichtung nach einem der vorstehenden Ansprüche, **dadurch gekennzeichnet, dass** der Laser (34) ein Impulslaser ist, der konfiguriert ist, um einen Strahl zu erzeugen, dessen Energie im Bereich zwischen 5 mJ/Impuls und 12 mJ/Impuls liegt, beispielsweise zwischen 5 mJ/Impuls und 10 mJ/Impuls, und dessen Oberfläche im Bereich zwischen 30 µm² und 3 mm² liegt.

6. Vorrichtung nach einem der Ansprüche 4 oder 5, **dadurch gekennzeichnet, dass** der Laser (34) konfiguriert ist, um einen Strahl zu erzeugen, dessen Oberfläche im Bereich zwischen 0,5 mm² und 2 mm² liegt.

7. Vorrichtung nach einem der vorstehenden Ansprüche, **dadurch gekennzeichnet, dass** ein System (42) zur Fokussierung und/oder Übertragung, hierbei von Molekülionen, die bei einer Abtragung von biologischem Material gebildet werden sollen, zwischen dem Übertragungsrohr (T, 21) und dem Massenspektrometer (31) angeordnet ist.

8. Vorrichtung nach dem vorstehenden Anspruch, **dadurch gekennzeichnet, dass** das System (42) zur Fokussierung und/oder Übertragung eine Übertragungskapillare ist.

9. Vorrichtung nach einem der vorstehenden Ansprüche, **dadurch gekennzeichnet, dass** das Übertragungsrohr (T, 21) mit einem Heizmittel (44) versehen ist.

10. Vorrichtung nach einem der Ansprüche 6 bis 8, **dadurch gekennzeichnet, dass** eine Druckentlastungskapillare (41) zwischen dem Übertragungsrohr (T, 21) und dem System (42) zur Fokussierung und/oder Übertragung eingesetzt ist, wobei der Innendurchmesser dieser Druckentlastungskapillare (41) kleiner als der des Übertragungsrohrs (T, 21) ist.

11. Vorrichtung nach dem vorstehenden Anspruch, **dadurch gekennzeichnet, dass** die Druckentlastungskapillare (41) metallisch ist und dass ein Mittel zum Erzeugen einer Potentialdifferenz zwischen der Druckentlastungskapillare (41) und dem Massenspektrometer (31) vorgesehen ist.

12. Vorrichtung nach einem der vorstehenden Ansprüche, **dadurch gekennzeichnet, dass** ein Metallgitter (48) zwischen dem Übertragungsrohr (T, 21) und dem Massenspektrometer (31) eingeführt ist.

13. Vorrichtung nach einem der Ansprüche 10 bis 12, **dadurch gekennzeichnet, dass** eine Vernebelungskapillare (45) an die Druckentlastungskapillare (41) angeschlossen ist und überdies mit einem Lösungsmittelverteilungsmittel (46) verbunden ist.

14. Vorrichtung nach einem der Ansprüche 1 bis 12, **dadurch gekennzeichnet, dass** zwischen dem Übertragungsrohr (T, 21) und dem Massenspektrometer (31) keine Vernebelungskapillare vorgesehen ist, die mit einem Lösungsmittelverteilungsmittel verbunden ist.

15. Vorrichtung nach einem der Ansprüche 1 bis 13, **dadurch gekennzeichnet, dass** eine Verteilungskapillare an die Druckentlastungskapillare angeschlossen und überdies mit einem Gasverteiler verbunden ist.

16. Vorrichtung nach einem der Ansprüche 1 bis 14, **dadurch gekennzeichnet, dass** zwischen dem Übertragungsrohr (T, 21) und dem Massenspektrometer (31) keine Verteilungskapillare vorgesehen ist, die mit einem Gasverteiler verbunden ist.

17. Vorrichtung nach einem der vorstehenden Ansprüche, **dadurch gekennzeichnet, dass** die Sonde (S, 10) eine zweite Analysefaser (15) aufweist.

18. Vorrichtung nach einem der vorstehenden Ansprüche, **dadurch gekennzeichnet, dass** die Sonde (S, 10) außerdem eine Therapiefaser (16) aufweist.

19. Vorrichtung nach dem vorstehenden Anspruch, **dadurch gekennzeichnet, dass** die Therapiefaser (16) an einen Therapielaser (36) angeschlossen ist.

20. Vorrichtung nach einem der vorstehenden Ansprüche, **dadurch gekennzeichnet, dass** die Sonde (S, 10) außerdem einen Beleuchtungskanal (12) und einen Bildaufnahmekanal (13) aufweist.

## Claims

1. A molecular analysis device for a biological material comprises:
- a laser (34), optionally assisted with an optical parametric oscillator (OPO), **configured for emitting a wavelength comprised between 2.5 µm and 12 µm, said** thereby configured laser (34) being intended to ablate said biological material by ejecting charged and/or non-charged particles;
- a mass spectrometer (31); and
- a probe (S, 10) including:
∘ at least one first analysis fiber (A, 14), and
∘ a transfer tube (T, 21),
said at least one first analysis fiber (A, 14) being connected to the laser and said transfer tube being connected to the mass spectrometer (31).

2. The device according to claim 1, **characterized in that** the laser (34), optionally assisted with an optical parametric oscillator (OPO) is configured for emitting a wavelength **comprised between 2.5 µm and 3.5 µm.**

3. The device according to any of the preceding claims, **characterized in that** the laser (34), optionally assisted with an optical parametric oscillator (OPO), is configured for **emitting a wavelength comprised between 2.8 µm and 3.2 µm.**

4. The device according to any of the preceding claims, **characterized in that** the laser (34) is a pulsed laser configured for generating a beam for which the energy is comprised between 2 mJ/pulse and 15 mJ/pulse and the surface of which is comprised between 30 µm² and 3 mm².

5. The device according to any of the preceding claims, **characterized in that** the laser (34) is a pulsed laser configured for generating a beam for which the energy is comprised between 5 mJ/pulse and 12 mJ/pulse, for example between 5 mJ/pulse and 10 mJ/pulse and **the surface is comprised between 30 µm²** and 3 mm².

6. The device according to one of claims 4 or 5, **characterized in that** the laser (34) is a configured for generating a beam for which the surface area is comprised between 0.5 mm² and 2 mm².

7. The device according to one of the preceding claims, **characterized in that** a system (42) for focussing and/or transferring, molecular ions intended to be formed during ablation of biological material, is interposed between said transfer tube (T, 21) and the mass spectrometer (31).

8. The device according to the preceding claim, **characterized in that** said focussing and/or transfer system (42) is a transfer capillary.

9. The device according to any of the preceding claims, **characterized in that** said transfer tube (T, 21) is provided with a heating means (44).

10. The device according to any of claims 6 to 8, **characterized in that** a de-pressurization capillary (41) is inserted between said transfer tube (T, 21) and said focussing and/or transfer system (42), the inner diameter of this de-pressurization capillary (41) being less than that of the transfer tube (T, 21).

11. The device according to the preceding claim, **characterized in that** the de-pressurization capillary (41) is a metal capillary and **in that** a means is provided for generating a potential difference between said de-pressurization capillary (41) and the mass spectrometer (31).

12. The device according to any of the preceding claims, **characterized in that** a metal grid (48) is introduced between said transfer tube (T, 21) and said mass spectrometer (31).

13. The device according to any of claims 10 to 12, **characterized in that** an electrospray capillary (45) is connected to said de-pressurization capillary (41) and further connected to a means for distributing solvent (46).

14. The device according to any of claims 1 to 12, **characterized in that** no electrospray capillary connected to a means for distributing solvent is provided between the transfer tube (T, 21) and the mass spectrometer (31).

15. The device according to any of claims 1 to 13, **characterized in that** a distribution capillary is connected to said de-pressurization capillary and further connected to a gas distributor.

16. The device according to one of claims 1 to 14, **characterized in that** no distribution capillary connected to a gas distributor is provided between the transfer tube (T, 21) and the mass spectrometer (31).

17. The device according to any of the preceding claims, **characterized in that** said probe (S, 10) includes a second analysis fiber (15).

18. The device according to any of the preceding claims, **characterized in that** said probe (S, 10) further includes a therapy fiber (16).

19. The device according to the preceding claim, **characterized in that** said therapy fiber (16) is connected to a therapy laser (36).

20. The device according to any of the preceding claims, **characterized in that** said probe (S, 10) further includes an illumination channel (12) and an image shooting channel (13).
